# EUROPÄISCHE PATENTSCHRIFT

(11) **EP 2 165 700 B1**
(45) Veröffentlichungstag und Bekanntmachung des Hinweises auf die Patenterteilung: **30.09.2015**
(21) Anmeldenummer: 09169003.2
(22) Anmeldetag: 31.08.2009
(51) Int. Cl.: C08G 18/42, A61K 8/87, A61Q 5/00, A41G 5/00

(54) **HAARVERLÄNGERUNG UND/ODER HAARVERDICHTUNGEN**
HAIR EXTENSION AND/OR HAIR VOLUME ATTACHMENTS
ALLONGEMENT ET/OU ÉPAISSISSEMENT DES CHEVEUX

(30) Priorität: 22.09.2008 DE 102008048391
(43) Veröffentlichungstag der Anmeldung: 24.03.2010
(73) Patentinhaber: Henkel AG & Co. KGaA, 40589 Düsseldorf (DE)
(72) Erfinder: Schulze Zur Wiesche, Erik, 20144, Hamburg (DE); Scheunemann, Volker, 21339, Lüneburg (DE); Westphal, Petra, 21629, Neu Wulmstorf (DE); Kasper, Dirk, 40597, Düsseldorf (DE)

(56) Entgegenhaltungen:
- EP-A1- 0 934 960
- EP-A1- 1 642 510
- DE-A1- 19 626 107
- DE-A1-102006 047 949
- DE-U1- 20 203 301

## Beschreibung

Die vorliegende Erfindung betrifft Haarsträhnen und Methoden zur Haarverlängerung und/oder Haarverdichtung.

Die Veränderung von Form oder Farbe des Kopfhaares zur Veränderung des Erscheinungsbildes ist allgemein bekannt und weit verbreitet. Kurzzeitiges Styling durch Haargele oder Haarsprays und länger anhaltende Stylings wie Dauerwellen sowie die Färbung oder Blondierung sind gesicherter Stand technischen Wissens und können zum großen Teil auch in Heimanwendung durchgeführt werden.

Ein weiteres Feld der Veränderung des Erscheinungsbildes hat sich in den letzten Jahren herausgebildet: Die Haarverlängerung. Hierbei handelt es sich um verschiedene Methoden, das Eigenhaar am Kopf durch fremde Echt- oder Kunsthaarsträhnen länger erscheinen zu lassen.

Das fremde Haar wird dabei strähnenweise mit den eigenen Haaren fest verbunden. Dies kann durch Wärme, Ultraschall oder mechanisch erfolgen. Eine weitere Möglichkeit ist die Einarbeitung von Tressen, bei der reihenweise gearbeitet wird. Die Befestigung findet vor allem am Hinterkopf statt, damit genügend Eigenhaar vom Oberkopf über die Verbindungsstellen fällt.

Hierbei existieren verschiedene Methoden:
- Bei der Bellargo-Methode werden Strähnen aus europäischem unbehandeltem Schnitthaar mit Hilfe von winzigen Hülsen durch Wärmeeinwirkung am Eigenhaar befestigt.
- Bei der Klebe- oder Bonding-Methode werden vorbehandelte Strähnen aus europäischem oder indischem Naturhaar an das Eigenhaar angeklebt. (unter Wärmeeinwirkung)
- Bei der Air-Pressure-Methode werden bis zu zehn Strähnen aus Echthaar gleichzeitig befestigt.
- Bei der Metallhülsen-Methode wird das Fremdhaar - meist indisches Naturhaar - mit einer speziellen Zange zusammengedrückt.
- Bei der Kunsthaar-Methode werden Kunstfasern mittels eines Thermoklebers in der Nähe der Kopfhaut befestigt Kunsthaare werden auch mit Metallspangen eingeklemmt.

Beim Anmodulieren mit Wärme wird Thermoklebstoff, Wachs, Keratin oder eine Silikonbasis bei ca. 90 - 130 °C benutzt, bei Ultraschall werden die Karatinplättchen (so genannte Bondings) auf molekularer Ebene kurzzeitig erwärmt, mechanische Methoden mit Plastik- oder Metallhülsen arbeiten ohne Wärme.

Eine Haarverlängerung hält bis zu 6 Monate, die Kosten variieren stark je nach Qualität der eingesetzten Haare und der angewendeten Einarbeitungsmethode.

Es sind andere Verfahren zur Herstellung einer Verbindung zwischen den Haarsträhnen und dem Eigenhaar bekannt, die externe Elemente, wie Spangen, Kämme, Klemmen usw. verwenden: Diese Elemente können dazu verwendet werden, Haarsträhnen mit größeren Abmessungen an das Haar des Anwenders zu befestigen, sie haben aber den großen Nachteil, dass ihre Verbindung deutlich sichtbar und durch den Anwender fühlbar ist, der dies als störend empfinden kann.

Beispiele für Haarverlängerungsverfahren sind aus den Druckschriften JP 03152205 (Aderans Co. Ltd.) und in der ZA 93/5214 A oder in der DE 196 26 107 C1 beschrieben. Verfahren, bei denen ein Klebstoff auf die entstehende Verbindung aufgebracht wird, sind beispielsweise in der US 4,934,387 (Megna) beschrieben. Ein Verfahren zur Befestigung einer selbsthaftenden Haarsträhne ist in der US-A-6,135,122 offenbart. Die DE 202 03 301 U1 offenbart schließlich eine Vorrichtung und ein Verfahren, bei dem mehrere Haarsträhnen mit Klebestellen an einer gemeinsamen Trägerfolie vorliegen und so gleichzeitig appliziert werden können.

Die DE 10 2006 047 949 A1 offenbart Vorrichtungen zur Haarverlängerung, wobei Enden von Haarsträhnen aus natürlichem Haar oder Kunsthaar in Polyurethan eingegossen werden. Die EP 1 759 604 A1 offenbart Vorrichtungen zur Haarverlängerung, wobei Enden von Haarsträhnen aus natürlichem Haar oder Kunsthaar mittels Perforierung in Polyurethanstreifen eingefügt werden. Beide Vorrichtungen können dann mittels Klebeband am natürlichen Kopfhaar befestigt werden.

Der vorliegenden Erfindung lag die Aufgabe zugrunde, das Gebiet der anklebbaren Haarsträhnen weiterzuentwickeln und insbesondere die Applikationszeiten zu verkürzen und das spätere Entfernen der Strähnen zu vereinfachen.

Es wurde nun gefunden, daß sich bestimmte Klebstoffe besonders zur Lösung dieser Aufgabe eignen und die Applikationsmöglichkeiten deutlich erweitern, so daß nicht mehr zwingend mit Wärme gearbeitet werden muß.

Gegenstand der vorliegenden Erfindung ist in einer ersten Ausfuhrungsform eine Vorrichtung zur Haarverlängerung, umfassend ein Haarteil aus jeweils mehreren Natur- und/oder Kunsthaaren sowie ein am Haarteil angebrachtes Befestigungselement, dadurch gekennzeichnet, daß das Befestigungselement mindestens ein themoplastisches Polyurethan-Elastomer umfaßt, das zusammengesetzt ist aus:
a) mindestens einem Polyisocyanat,
b) mindestens einem Polyesterglykol.

Die erfindungsgemäße Vorrichtung besteht aus einem Haarteil, an dem ein Befestigungselement angebracht ist. Das Haarteil besteht aus einer Mehrzahl von natürlichen und/oder künstlichen Fasern ("Haarsträhne"). Natürliche ("echte") Haare stammen oft aus Indien oder China, da hier Frisuren oft traditionell lang sind. In Indien werden die Zöpfe teilweise aus religiösen Motiven abgeschnitten. Heute stammen die meisten Menschenhaare aus Indien. Auch Tierhaare können erfindungsgemäß eingesetzt werden, bewährt hat sich hierbei beispielsweise Büffelbauchhaar. Die Haare werden gewaschen, nach Länge sortiert, gekraust und gefärbt. Oft wird auch die äußere Schuppenschicht entfernt um ein Filzen zu verhindern.

Erfindungsgemäße Vorrichtungen können auch aus Kunsthaar hergestellt werden. Ein weit verbreitetes Material sind Kunstfasern aus Poly-(acrylnitril-co-vinylchlorid) von der Firma Kanekalon. Auch Strähnen, in denen sowohl natürliche Haare als auch Kunstfasern nebeneinander vorliegen, sind erfindungsgemäß geeignet..

Am Haarteil befindet sich ein Befestigungselement, das vorzugsweise an einem Ende der Haarsträhne angebracht wird. Ein Anbringen des Befestigungselementes an anderen Stellen, beispielsweise in der Mitte einer Haarsträhne ist ebenfalls möglich - hierdurch wird aber ja nach Länge der Haarsträhne ein unnatürlicher Strähnenverlauf erzeugt. Vorzugsweise liegt das Befestigungselement daher an einem Ende (nachfolgend als "oberes Ende" bezeichnet) der Haarstsrähne vor. Das Befestigungselement umfaßt erfindungsgemäß mindestens ein themoplastisches Polyurethan-Elastomer, das zusammengesetzt ist aus: mindestens einem Polyisocyanat und mindestens einem Polyesterglykol.

Polyurethan ist die Sammelbezeichnung für Polymere, in deren Makromoleküle die Wiederholungseinheiten durch Urethan-Gruppierungen -NH-CO-O- verknüpft sind. Polyurethane werden im Allgemeinen erhalten durch Polyaddition aus zwei- oder höherwertigen Alkoholen und Isocyanaten gemäß

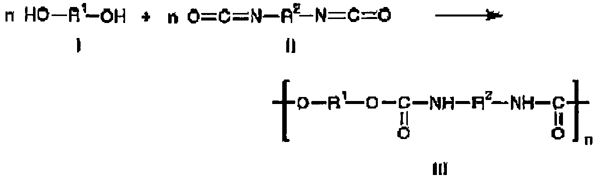

R¹ und R² können dabei für niedermolekulare oder selbst schon polymere aliphatische oder aromatische Gruppen stehen. Technisch wichtige Polyurethane werden hergestellt aus Polyester- und/oder Polyetherdiolen und z.B. 2,4- bzw. 2,6-Toluoldiisocyanat (TDI, R² = C₆H₃-CH₃), 4,4'-Methylenbis(phenylisocyanat) (MDI, R² = C₆H₄-CH₂-C₆H₄), 4,4'-Methylendicyclohexylisocyanat (HMDI, R² = C₆H₁₀-CH₂-C₆H₁₀) od. Hexamethylendiisocyanat [HDI, R² = (CH₂)₆].

Erfindungsgemäß umfaßt das Befestigungselement mindestens ein themoplastisches Polyurethan-Elastomer, das zusammengesetzt ist aus:
a) mindestens einem Polyisocyanat,
b) mindestens einem Polyesterglykol.

Unter einem "Polyisocyanat" ist eine niedermolekulare Verbindung mit 2 oder 3 Isocyanat-Gruppen zu verstehen. Die Diisocyanate werden bevorzugt, sie können aber bis zu ca. 10 Gew.-% an trifunktionellem Isocyanat enthalten. Neben aliphatischen und cycloaliphatischen Polyisocyanaten kommen vor allem aromatische Polyisocyanate in Frage. Konkrete Beispiele sind: Toluol-diisocyanat, Diphenylmethandiisocyanat und deren Mischungen. Unter Diphenylmethandiisocyanat wird sowohl das 4,4'- als auch das 2,4'-Diphenylmethandiisocyanat verstanden. Vorzugsweise sollte das 2,4'-losmere aber nicht mehr als 50 Gew.-% ausmachen. Es werden vorzugsweise ein oder zwei verschiedene Polyisocyanate eingesetzt. Vor allem wird reines 4,4'-Diphenylmethandiisacyanat verwendet. Der Anteil des Polyisocyanates im PU-Polymer sollte 15 bis 35, vorzugsweise 20 bis 30 Gew.-% betragen.

Ganz besonders bevorzugte erfindungsgemäße Vorrichtungen sind dadurch gekennzeichnet, daß das Polyurethan-Polymer als Monomer a) (Polyisocyanat) mindestens ein aromatisches Diisocyanat enthält, vorzugsweise ein Diisocyanat aus der Gruppe Toluoldiisocyanat, 4,4'-Diphenylmethandiisocyanat und 2,4'-Diphenylmethandiisocyanat sowie deren Mischungen.

Unter einem "Polyesterglykol" wird ein Polyester mit 2 OH-Gruppen verstanden, vorzugsweise mit 2 endständigen OH-Gruppen. Sie werden auf bekanntem Wege hergestellt, sei es aus
a) aliphatischen Hydroxycarbonsäuren oder aus
b) aliphatischen Dicarbonsäuren mit 6 bis 12 C-Atomen und - insbesondere geradzahligen - Diolen mit 4 bis 8 C-Atomen.
Natürlich können auch entsprechende Derivate eingesetzt werden, z. B Lactone, Methylester oder Anhydride. Konkrete Ausgangsprodukte sind: 1,4-Butandiol, 1,4-Hexandiol, Adipin-, Azelain-, Sebazinsäure und Lacton. Die Säurekomponente kann bis zu 25 Mol-% einer anderen Säure enthalten, z. B. Cyclohexandicarbonsäure, Terephthalsäure und Isophthalsäure. Die Glykolkomponente kann bis zu 15 Mol-% eines anderen Diols enthalten, z. B. Diethylenglykol, 1,4-Cyclohexandimethanol. Neben Homopolymeren aus obigen Bausteinen sind vor allem Copolyester aus den folgenden Bausteinen oder deren Derivaten wichtig:
1. Adipinsäure, isophthalsäure, Phthalsäure und Butandiol,
2. Adipinsäure, Phthalsäure und Hexandiol,
3. Adipinsäure, Isophthalsäure, Phthalsäure, Ethylenglykol, Neopentylglykol und 3-Hydroxy-2,2-dimethylpropyl-3-hydroxy-2,2-dimethylpropanoat und
4. Adipinsäure, Phthalsäure, Neopentylglykol und Ethylenglykol.

Der Copolyester aus Adipinsäure, Isophthalsäure, Phthalsäure und Butandiol ist teilweise kristallin und hat eine hohe Viskosität. Er führt daher zu hohen Anfangsfestigkeiten. Der Copolyester aus Adipinsäure, Phthalsäure und Hexandiol hat eine niedrige Glasübergangstemperatur und führt daher zu einer verbesserten Kälteflexibilität.

Die Polyesterglykole sind flüssig oder fest. Wenn sie fest sind, sind sie vorzugsweise amorph. Sie können aber auch schwachkristallin sein. Vorzugsweise wird eine Mischung aus teilweise kristallinen und amorphen Polyestern eingesetzt. Die Kristallinität ist allerdings so wenig ausgeprägt, daß sie sich in dem fertigen thermoplatischen Polyurethan-Elastomer nicht durch Trübung bemerkbar macht. Der Schmelzpunkt der teilkristallinen Polyester liegt vorzugsweise im Bereich von 40 bis 70 °C, besonders bevorzugt im Bereich von 45 bis 65 °C. Der Schmelzpunkt gibt die Temperatur an, bei der die kristallinen Bereiche des Materials schmelzen. Er wird differential-thermoanalytisch bestimmt durch den Endotherm-Hauptpeak. Vorzugsweise wird ein Polybutandioladipat mit einem Molekulargewicht von etwa 3 500 und einem Schmelzpunkt von etwa 50 °C als teilweise kristallines Polyesterglykol verwendet.

Ganz besonders bevorzugte erfindungsgemäße Vorrichtungen sind dadurch gekennzeichnet, daß das thermoplatische Polyurethan-Elastomer als Monomer b) (Polyesterglycol) mindestens eine Verbindung der Formel PEG-I) 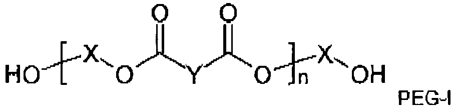 enthält, in der
- X für -(Ch₂)ₘ- mit m = 2, 3, 4, 5, 6 oder für -(CH₂)ₖ-(C₆H₄)-(CH₂)ₖ- mit k = 1, 2, 3, 4, 5, 6 steht,
- Y für -(CH₂)ₘ- mit m = 0, 1, 2, 3, 4, 5, 6, 7, 8, 9, 10, 11, 12, 14, 16 oder für -(CH₂)ₖ-(C₆H₄)-(CH₂)ₖ- mit k= 1, 2, 3, 4, 5, 6 steht
- n eine ganze Zahl ist.

Die Verbindungen der Formel (PEG-I) sind Ester von linearen Dicarbonsäuren mit linearen Diolen. Besonders bevorzugte erfindungsgemäß einsetzbare Polyesterpolyole der Formel (PEG-I), in denen n jeweils für eine ganze Zahl steht, sind in der nachstehenden Tabelle aufgeführt:

| **X** | **Y** |
|---|---|
| -(CH₂)₂- | -(CH₂)- |
| -(CH₂)₂- | -(CH₂)₂- |
| -(CH₂)₂- | -(CH₂)₃- |
| -(CH₂)₂- | -(CH₂)₄- |
| -(CH₂)₂- | -(CH₂)₅- |
| -(CH₂)₂- | -(CH₂)₆- |
| -(CH₂)₂- | -(CH₂)₇- |
| -(CH₂)₂- | -(CH₂)₈- |
| -(CH₂)₂- | -(CH₂)₉- |
| -(CH₂)₂- | -(CH₂)₁₀- |
| -(CH₂)₂- | -(CH₂)₁₁- |
| -(CH₂)₂- | -(CH₂)₁₂- |
| -(CH₂)₂- | -(CH₂)₁₄- |
| -(CH₂)₂- | -(CH₂)₁₆- |
| -(CH₂)₂- | -(CH₂)-(C₆H₄)-(CH₂)- |
| -(CH₂)₂- | -(CH₂)₂-(C₆H₄)-(CH₂)₂- |
| -(CH₂)₂- | -(CH₂)₃-(C₆H₄)-(CH₂)₃- |
| -(CH₂)₂- | -(CH₂)₄-(C₆H₄)-(CH₂)₄- |
| -(CH₂)₂- | -(CH₂)₅-(C₆H₄)-(CH₂)₅- |
| -(CH₂)₂- | -(CH₂)₆-(C₆H₄)-(CH₂)₆- |
| -(CH₂)₃- | -(CH₂)- |
| -(CH₂)₃- | -(CH₂)₂- |
| -(CH₂)₃- | -(CH₂)₃- |
| -(CH₂)₃- | -(CH₂)₄- |
| -(CH₂)₃- | -(CH₂)₅- |
| -(CH₂)₃- | -(CH₂)₆- |
| -(CH₂)₃- | -(CH₂)₇- |
| -(CH₂)₃- | -(CH₂)₈- |
| -(CH₂)₃- | -(CH₂)₉- |
| -(CH₂)₃- | -(CH₂)₁₀- |
| -(CH₂)₃- | -(CH₂)₁₁- |
| -(CH₂)₃- | -(CH₂)₁₂- |
| -(CH₂)₃- | -(CH₂)₁₄- |
| -(CH₂)₃- | -(CH₂)₁₆- |
| -(CH₂)₃- | -(CH₂)-(C₆H₄)-(CH₂)- |
| -(CH₂)₃- | -(CH₂)₂-(C₆H₄)-(CH₂)₂- |
| -(CH₂)₃- | -(CH₂)₃-(C₆H₄)-(CH₂)₃- |
| -(CH₂)₃- | -(CH₂)₄-(C₆H₄)-(CH₂)₄- |
| -(CH₂)₃- | -(CH₂)₅-(C₆H₄)-(CH₂)₅- |
| -(CH₂)₃- | -(CH₂)₆-(C₆H₄)-(CH₂)₆- |
| -(CH₂)₄- | -(CH₂)- |
| -(CH₂)₄- | -(CH₂)₂- |
| -(CH₂)₄- | -(CH₂)₃- |
| -(CH₂)₄- | -(CH₂)₄- |
| -(CH₂)₄- | -(CH₂)₅- |
| -(CH₂)₄- | -(CH₂)₆- |
| -(CH₂)₄- | -(CH₂)₇- |
| -(CH₂)₄- | -(CH₂)₈- |
| -(CH₂)₄₋ | -(CH₂)₉- |
| -(CH₂)₄- | -(CH₂)₁₀- |
| -(CH₂)₄- | -(CH₂)₁₁- |
| -(CH₂)₄- | -(CH₂)₁₂- |
| -(CH₂)₄- | -(CH₂)₁₄- |
| -(CH₂)₄- | -(CH₂)₁₆- |
| -(CH₂)₄- | -(CH₂)-(C₆H₄)-(CH₂)- |
| -(CH₂)₄- | -(CH₂)₂-(C₆H₄)-(CH₂)₂- |
| -(CH₂)₄- | -(CH₂)₃-(C₆H₄)-(CH₂)₃- |
| -(CH₂)₄- | -(CH₂)₄-(C₆H₄)-(CH₂)₄- |
| -(CH₂)₄- | -(CH₂)₅-(C₆H₄)-(CH₂)₅- |
| -(CH₂)₄- | -(CH₂)₆-(C₆H₄)-(CH₂)₆- |
| -(CH₂)₅- | -(CH₂)- |
| -(CH₂)₅- | -(CH₂)₂- |
| -(CH₂)₅- | -(CH₂)₃- |
| -(CH₂)₅- | -(CH₂)₄- |
| -(CH₂)₅- | -(CH₂)₅- |
| -(CH₂)₅- | -(CH₂)₆- |
| -(CH₂)₅- | -(CH₂)₇- |
| -(CH₂)₅- | -(CH₂)₈- |
| -(CH₂)₅- | -(CH₂)₉- |
| -(CH₂)₅- | -(CH₂)₁₀- |
| -(CH₂)₅- | -(CH₂)₁₁- |
| -(CH₂)₅- | -(CH₂)₁₂- |
| -(CH₂)₅- | -(CH₂)₁₄- |
| -(CH₂)₅- | -(CH₂)₁₆- |
| -(CH₂)₅- | -(CH₂)-(C₆H₄)-(CH₂)- |
| -(CH₂)₅- | -(CH₂)₂-(C₆H₄)-(CH₂)₂- |
| -(CH₂)₅- | -(CH₂)₃-(C₆H₄)-(CH₂)₃- |
| -(CH₂)₅- | -(CH₂)₄-(C₆H₄)-(CH₂)₄- |
| -(CH₂)₅- | -(CH₂)₅-(C₆H₄)-(CH₂)₅- |
| -(CH₂)₅- | -(CH₂)₆-(C₆H₄)-(CH₂)₆- |
| -(CH₂)₆- | -(CH₂)- |
| -(CH₂)₆- | -(CH₂)₂- |
| -(CH₂)₆- | -(CH₂)₃- |
| -(CH₂)₆- | -(CH₂)₄- |
| -(CH₂)₆- | -(CH₂)₅- |
| -(CH₂)₆- | -(CH₂)₆- |
| -(CH₂)₆- | -(CH₂)₇- |
| -(CH₂)₆- | -(CH₂)₈- |
| -(CH₂)₆- | -(CH₂)₉- |
| -(CH₂)₆- | -(CH₂)₁₀- |
| -(CH₂)₆- | -(CH₂)₁₁- |
| -(CH₂)₆- | -(CH₂)₁₂- |
| -(CH₂)₆- | -(CH₂)₁₄- |
| -(CH₂)₆- | -(CH₂)₁₆- |
| -(CH₂)₆- | -(CH₂)-(C₆H₄)-(CH₂)- |
| -(CH₂)₆- | -(CH₂)₂-(C₆H₄)-(CH₂)₂- |
| -(CH₂)₆- | -(CH₂)₃-(C₆-H₄)-(CH₂)₃- |
| -(CH₂)₆- | -(CH₂)₄(C₆H₄)-(CH₂)₄- |
| -(CH₂)₆- | -(CH₂)₅-(C₆H₄)-(CH₂)₅- |
| -(CH₂)₆- | -(CH₂)₆(C₆H₄)-(CH₂)₆- |
| -(CH₂)-(C₆H₄)-(CH₂)- | -(CH₂)- |
| -(CH₂)-(C₆H₄)-(CH₂)- | -(CH₂)₂- |
| -(CH₂)-(C₆H₄)-(CH₂)- | -(CH₂)₃- |
| -(CH₂)-(C₆H₄)-(CH₂)- | -(CH₂)₄- |
| -(CH₂)-(C₆H₄)-(CH₂)- | -(CH₂)₅- |
| -(CH₂)-(C₆H₄)-(CH₂)- | -(CH₂)₆- |
| -(CH₂)-(C₆H₄)-(CH₂)- | -(CH₂)₇- |
| -(CH₂)-(C₆H₄)-(CH₂)- | -(CH₂)₈- |
| -(CH₂)-(C₆H₄)-(CH₂)- | -(CH₂)₉- |
| -(CH₂)-(C₆H₄)-(CH₂)- | -(CH₂)₁₀- |
| -(CH₂)-(C₆H₄)-(CH₂)- | -(CH₂)₁₁- |
| -(CH₂)-(C₆H₄)-(CH₂)- | -(CH₂)₁₂- |
| -(CH₂)-(C₆H₄)-(CH₂)- | -(CH₂)₁₄- |
| -(CH₂)-(C₆H₄)-(CH₂)- | -(CH₂)₁₆- |
| -(CH₂)-(C₆H₄)-(CH₂)- | -(CH₂)-(C₆H₄)-(CH₂)- |
| -(CH₂)-(C₆H₄)-(CH₂)- | -(CH₂)₂-(C₆H₄)-(CH₂)₂- |
| -(CH₂)-(C₆H₄)-(CH₂)- | -(CH₂)₃-(C₆H₄)-(CH₂)₃- |
| -(CH₂)-(C₆H₄)-(CH₂)- | -(CH₂)₄-(C₆H₄)-(CH₂)₄- |
| -(CH₂)-(C₆H₄)-(CH₂)- | -(CH₂)₅-(C₆H₄)-(CH₂)₅- |
| -(CH₂)-(C₆H₄)-(CH₂)- | -(CH₂)₆-(C₆H₄)-(CH₂)₆- |
| -(CH₂)₂-(C₆H₄)-(CH₂)₂- | -(CH₂)- |
| -(CH₂)₂-(C₆H₄)-(CH₂)₂- | -(CH₂)₂- |
| -(CH₂)₂-(C₆H₄)-(CH₂)₂- | -(CH₂)₃- |
| -(CH₂)₂-(C₆H₄)-(CH₂)₂- | -(CH₂)₄- |
| -(CH₂)₂-(C₆H₄)-(CH₂)₂- | -(CH₂)₅- |
| -(CH₂)₂-(C₆H₄)-(CH₂)₂- | -(CH₂)₆- |
| -(CH₂)₂-(C₆H₄)-(CH₂)₂- | -(CH₂)₇- |
| -(CH₂)₂-(C₆H₄)-(CH₂)₂- | -(CH₂)₈- |
| -(CH₂)₂-(C₆H₄)-(CH₂)₂- | -(CH₂)₉- |
| -(CH₂)₂-(C₆H₄)-(CH₂)₂- | -(CH₂)₁₀- |
| -(CH₂)₂-(C₆H₄)-(CH₂)₂- | -(CH₂)₁₁- |
| -(CH₂)₂-(C₆H₄)-(CH₂)₂- | -(CH₂)₁₂- |
| -(CH₂)₂-(C₆H₄)-(CH₂)₂- | -(CH₂)₁₄- |
| -(CH₂)₂-(C₆H₄)-(CH₂)₂- | -(CH₂)₁₆- |
| -(CH₂)₂-(C₆H₄)-(CH₂)₂- | -(CH₂)-(C₆H₄)-(CH₂)- |
| -(CH₂)₂-(C₆H₄)-(CH₂)₂- | -(CH₂)₂-(C₆H₄)-(CH₂)₂- |
| -(CH₂)₂-(C₆H₄)-(CH₂)₂- | -(CH₂)₃-(C₆H₄)-(CH₂)₃- |
| -(CH₂)₂-(C₆H₄)-(CH₂)₂- | -(CH₂)₄-(C₆H₄)-(CH₂)₄- |
| -(CH₂)₂-(C₆H₄)-(CH₂)₂- | -(CH₂)₅-(C₆H₄)-(CH₂)₅- |
| -(CH₂)₂-(C₆H₄)-(CH₂)₂- | -(CH₂)₆-(C₆H₄)-(CH₂)₆- |
| -(CH₂)₃-(C₆H₄)-(CH₂)₃- | -(CH₂)- |
| -(CH₂)₃-(C₆H₄)-(CH₂)₃- | -(CH₂)₂- |
| -(CH₂)₃-(C₆H₄)-(CH₂)₃- | -(CH₂)₃- |
| -(CH₂)₃-(C₆H₄)-(CH₂)₃- | -(CH₂)₄- |
| -(CH₂)₃-(C₆H₄)-(CH₂)₃- | -(CH₂)₅- |
| -(CH₂)₃-(C₆H₄)-(CH₂)₃- | -(CH₂)₆- |
| -(CH₂)₃-(C₆H₄)-(CH₂)₃- | -(CH₂)₇- |
| -(CH₂)₃-(C₆H₄)-(CH₂)₃- | -(CH₂)₈- |
| -(CH₂)₃-(C₆H₄)-(CH₂)₃- | -(CH₂)₉- |
| -(CH₂)₃-(C₆H₄)-(CH₂)₃- | -(CH₂)₁₀- |
| -(CH₂)₃-(C₆H₄)-(CH₂)₃- | -(CH₂)₁₁- |
| -(CH₂)₃-(C₆H₄)-(CH₂)₃- | -(CH₂)₁₂- |
| -(CH₂)₃-(C₆H₄)-(CH₂)₃- | -(CH₂)₁₄- |
| -(CH₂)₃-(C₆H₄)-(CH₂)₃- | -(CH₂)₁₆- |
| -(CH₂)₃-(C₆H₄)-(CH₂)₃- | -(CH₂)-(C₆H₄)-(CH₂)- |
| -(CH₂)₃-(C₆H₄)-(CH₂)₃- | -(CH₂)₂-(C₆H₄)-(CH₂)₂- |
| -(CH₂)₃-(C₆H₄)-(CH₂)₃- | -(CH₂)₃-(C₆H₄)-(CH₂)₃- |
| -(CH₂)₃-(C₆H₄)-(CH₂)₃- | -(CH₂)₄-(C₆H₄)-(CH₂)₄- |
| -(CH₂)₃-(C₆H₄)-(CH₂)₃- | -(CH₂)₅-(C₆H₄)-(CH₂)₅- |
| -(CH₂)₃-(C₆H₄)-(CH₂)₃- | -(CH₂)₆-(C₆H₄)-(CH₂)₆- |
| -(CH₂)₄-(C₆H₄)-(CH₂)₄- | -(CH₂)- |
| -(CH₂)₄-(C₆H₄)-(CH₂)₄- | -(CH₂)₂- |
| -(CH₂)₄-(C₆H₄)-(CH₂)₄- | -(CH₂)₃- |
| -(CH₂)₄-(C₆H₄)-(CH₂)₄- | -(CH₂)₄- |
| -(CH₂)₄-(C₆H₄)-(CH₂)₄- | -(CH₂)₅- |
| -(CH₂)₄-(C₆H₄)-(CH₂)₄- | -(CH₂)₆- |
| -(CH₂)₄-(C₆H₄)-(CH₂)₄- | -(CH₂)₇- |
| -(CH₂)₄-(C₆H₄)-(CH₂)₄- | -(CH₂)₈- |
| -(CH₂)₄-(C₆H₄)-(CH₂)₄- | -(CH₂)₉- |
| -(CH₂)₄-(C₆H₄)-(CH₂)₄- | -(CH₂)₁₀- |
| -(CH₂)₄-(C₆H₄)-(CH₂)₄- | -(CH₂)₁₁- |
| -(CH₂)₄-(C₆H₄)-(CH₂)₄- | -(CH₂)₁₂- |
| -(CH₂)₄-(C₆H₄)-(CH₂)₄- | -(CH₂)₁₄- |
| -(CH₂)₄-(C₆H₄)-(CH₂)₄- | -(CH₂)₁₆- |
| -(CH₂)₄-(C₆H₄)-(CH₂)₄- | -(CH₂)-(C₆H₄)-(CH₂)- |
| -(CH₂)₄-(C₆H₄)-(CH₂)₄- | -(CH₂)₂-(C₆H₄)-(CH₂)₂- |
| -(CH₂)₄-(C₆H₄)-(CH₂)₄- | -(CH₂)₃-(C₆H₄)-(CH₂)₃- |
| -(CH₂)₄-(C₆H₄)-(CH₂)₄- | -(CH₂)₄-(C₆H₄)-(CH₂)₄- |
| -(CH₂)₄-(C₆H₄)-(CH₂)₄- | -(CH₂)₅-(C₆H₄)-(CH₂)₅- |
| -(CH₂)₄-(C₆H₄)-(CH₂)₄- | -(CH₂)₆-(C₆H₄)-(CH₂)₆- |
| -(CH₂)₅-(C₆H₄)-(CH₂)₅- | -(CH₂)- |
| -(CH₂)₅-(C₆H₄)-(CH₂)₅- | -(CH₂)₂- |
| -(CH₂)₅-(C₆H₄)-(CH₂)₅- | -(CH₂)₃- |
| -(CH₂)₅-(C₆H₄)-(CH₂)₅- | -(CH₂)₄- |
| -(CH₂)₅-(C₆H₄)-(CH₂)₅- | -(CH₂)₅- |
| -(CH₂)₅-(C₆H₄)-(CH₂)₅- | -(CH₂)₆- |
| -(CH₂)₅-(C₆H₄)-(CH₂)₅- | -(CH₂)₇- |
| -(CH₂)₅-(C₆H₄)-(CH₂)₅- | -(CH₂)₈- |
| -(CH₂)₅-(C₆H₄)-(CH₂)₅- | -(CH₂)₉- |
| -(CH₂)₅-(C₆H₄)-(CH₂)₅- | -(CH₂)₁₀- |
| -(CH₂)₅-(C₆H₄)-(CH₂)₅- | -(CH₂)₁₁- |
| -(CH₂)₅-(C₆H₄)-(CH₂)₅- | -(CH₂)₁₂- |
| -(CH₂)₅-(C₆H₄)-(CH₂)₅- | -(CH₂)₁₄- |
| -(CH₂)₅-(C₆H₄)-(CH₂)₅- | -(CH₂)₁₆- |
| -(CH₂)₅-(C₆H₄)-(CH₂)₅- | -(CH₂)-(C₆H₄)-(CH₂)- |
| -(CH₂)₅-(C₆H₄)-(CH₂)₅- | -(CH₂)₂-(C₆H₄)-(CH₂)₂- |
| -(CH₂)₅-(C₆H₄)-(CH₂)₅- | -(CH₂)₃-(C₆H₄)-(CH₂)₃- |
| -(CH₂)₅-(C₆H₄)-(CH₂)₅- | -(CH₂)₄-(C₆H₄)-(CH₂)₄- |
| -(CH₂)₅-(C₆H₄)-(CH₂)₅- | -(CH₂)₅-(C₆H₄)-(CH₂)₅- |
| -(CH₂)₅-(C₆H₄)-(CH₂)₅- | -(CH₂)₆(C₆H₄)-(CH₂)₆- |
| -(CH₂)₆-(C₆H₄)-(CH₂)₆- | -(CH₂)- |
| -(CH₂)₆-(C₆H₄)-(CH₂)₆- | -(CH₂)₂- |
| -(CH₂)₆-(C₆H₄)-(CH₂)₆- | -(CH₂)₃- |
| -(CH₂)₆-(C₆H₄)-(CH₂)₆- | -(CH₂)₄- |
| -(CH₂)₆-(C₆H₄)-(CH₂)₆- | -(CH₂)₅- |
| -(CH₂)₆-(C₆H₄)-(CH₂)₆- | -(CH₂)₆- |
| -(CH₂)₆(C₆H₄)-(CH₂)₆- | -(CH₂)₇- |
| -(CH₂)₆-(C₆H₄)-(CH₂)₆- | -(CH₂)₈- |
| -(CH₂)₆-(C₆H₄)-(CH₂)₆- | -(CH₂)₉- |
| -(CH₂)₆-(C₆H₄)-(CH₂)₆- | -(CH₂)₁₀- |
| -(CH₂)₆-(C₆H₄)-(CH₂)₆- | -(CH₂)₁₁- |
| -(CH₂)₆-(C₆H₄)-(CH₂)₆- | -(CH₂)₁₂- |
| -(CH₂)₆-(C₆H₄)-(CH₂)₆- | -(CH₂)₁₄- |
| -(CH₂)₆-(C₆H₄)-(CH₂)₆- | -(CH₂)₁₆- |
| -(CH₂)₆-(C₆H₄)-(CH₂)₆- | -(CH₂)-(C₆H₄)-(CH₂)- |
| -(CH₂)₆-(C₆H₄)-(CH₂)₆- | -(CH₂)₂-(C₆H₄)-(CH₂)₂- |
| -(CH₂)₆-(C₆H₄)-(CH₂)₆- | -(CH₂)₃-(C₆H₄)-(CH₂)₃- |
| -(CH₂)₆-(C₆H₄)-(CH₂)₆- | -(CH₂)₄-(C₆H₄)-(CH₂)₄- |
| -(CH₂)₆-(C₆H₄)-(CH₂)₆- | -(CH₂)₅-(C₆H₄)-(CH₂)₅- |
| -(CH₂)₆-(C₆H₄)-(CH₂)₆- | -(CH₂)₆-(C₆H₄)-(CH₂)₆- |

Auch Polyesterglycole aus Lactonen sind erfindungsgemäß mit Vorzug einsetzbar. Hier sind erfindungsgemäße Vorrichtungen bevorzugt, bei denen das thermoplatische Polyurethan-Elastomer als Monomer b) (Polyesterglycol) mindestens eine Verbindung der Formel PEG-II) 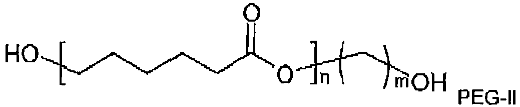 enthält, in der
- n für eine ganze Zahl steht,
- m für 2, 3, 4, 5, 6 steht

Erfindungsgemäß bevorzugte Polyesterglycole der Formel PEG-II sind demnach
- HO-[(CH₂)₆-C(O)-O-]ₙ(CH₂)₂-OH,
- HO-[(CH₂)₅-C(O)-O-]ₙ(CH₂)₃-OH,
- HO-[(CH₂)₅-C(O)-O-]ₙ(CH₂)₄-OH,
- HO-[(CH₂)₅-C(O)-O-]ₙ(CH₂)₅-OH,
- HO-[(CH₂)₅-C(O)-O-]ₙ(CH₂)₆-OH,
in denen n jeweils für eine ganze Zahl steht.

Unabhängig von der Art des Polyestergylcols (Formel PEG-I bzw. PEG-II) sollte das mittlere Molekulargewicht des Polyesterglykols (Mn) vorzugsweise zwischen 1 500 und 30 000 liegen, vorzugsweise zwischen 2 500 und 6 000. Es wird aus der OH-Zahl berechnet.

Erfindungsgemäß bevorzugte Vorrichtungen sind dadurch gekennzeichnet, daß die Molekulargewichte der Polyesterglykole zwischen 1 500 und 30 000, vorzugsweise zwischen 2 500 und 6 000 liegen.

Je nach Wahl des Diisocanats und des Polyesterglycols können Polyurethane mit unterschiedlichen Struktureinheiten gebildet werden. Erfindungsgemäß bevorzugte Vorrichtungen sind dadurch gekennzeichnet, daß das thermoplatische Polyurethan-Elastomer Struktureinheiten der Formel 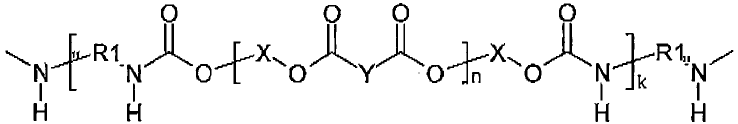 aufweist, in der
- X für -(CH₂)ₘ- mit m = 2, 3, 4, 5, 6 oder für -(CH₂)ₖ-(C₆H₄)-(CH₂)ₖ- mit k = 1, 2, 3, 4, 5, 6 steht,
- Y für -(CH₂)ₘ- mit m = 0, 1, 2, 3, 4, 5, 6, 7, 8, 9, 10, 11, 12, 14, 16 oder für -(CH₂)ₖ-(C₆H₄)-(CH₂)ₖ- mit k = 1, 2, 3, 4, 5, 6 steht
- R1 ausgewählt ist aus -(CH₂)₆-, 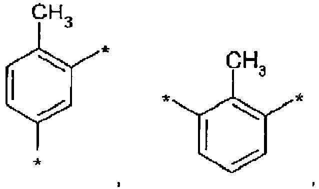 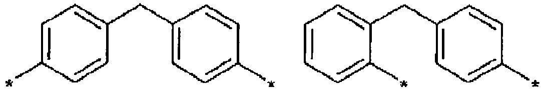
- n für eine ganze Zahl steht,
- k für eine ganze Zahl steht.

Setzt man nicht Polyester der Formel PEG-I mit Diisocyanaten um, sondern (auch) solche der Formel PEG-II, so gelangt man zu Polyurethanen, die (auch) andere Struktureinheiten aufweisen. Erfindungsgemäß besonders bevorzugt sind auch Vorrichtungen, bei denen das thermoplatische Polyurethan-Elastomer Struktureinheiten der Formel 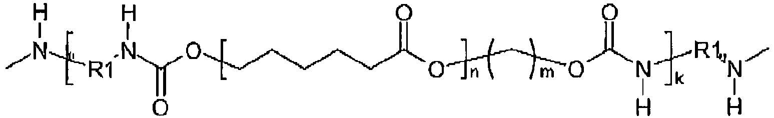 aufweist, in der
- R1 ausgewählt ist aus -(CH₂)₆-, 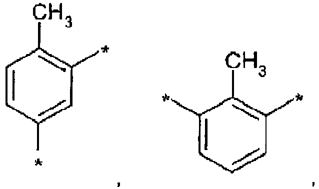 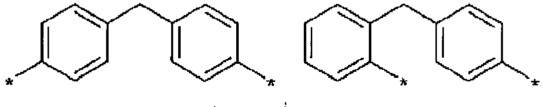
- n für eine ganze Zahl steht,
- m für 2, 3, 4, 5, 6 steht,
- k für eine ganze Zahl steht.

Die erfindungsgemäßen Vorrichtungen können Befestigungselemente umfassen, in denen das Polyurethan weitere Struktureinheiten enthält. Besonders bevorzugt sind beispielsweise Vorrichtungen, bei denen das thermoplatische Polyurethan-Elastomer zusätzlich mindestens eine Struktureinheit der Formel

-[O-(CH₂)ₙ-O]-

aufweist, in der n für 2, 3, 4, 5, oder 6 steht.

Diese Struktureinheiten werden erhalten, indem bei der Polyadditionsreaktion Diole zugegen sind, wobei bevorzugte Diole Ethandiol (n = 2), 1,3-Propandiol (n = 3), 1,4-Butandiol (n = 4) und 1,6-Hexandiol (n = 6) sind.

Vorzugsweise liegt die Viskosität der PU-Polymere in Lösung innerhalb bestimmter Bereiche. Hier sind erfindungsgemäße Vorrichtungen bevorzugt, die dadurch gekennzeichnet sind, daß das thermoplatische Polyurethan-Elastomer bei 20°C in 15 Gew.-%-iger Lösung in Methylethylketon (Brookfield-Viskosimeter RVF, Spindel 2, 20 U/min) Viskositäten von 1000 bis 2500 mPas, vorzugsweise von 1200 bis 2000 mPas und insbesondere von 1400 bis 1800 mPas, aufweist.

Die erfindungsgemaß eingesetzten thermoplatischen Polyurethan-Elastomere können ggf. weitere Zusatz- und/oder Füllstoffe enthalten. Falls die gewünschten Zusätze nicht bereits während der Bildung des Polyurethan-Polymers zugesetzt wurden, müssen sie bei der Verarbeitung des Polyurethans zum Befestigungselement zugesetzt und homogenisiert werden.

Es ist erfindungsgemäß bevorzugt, den thermoplatischen Polyurethan-Elastomeren keine Füllstoffe aus den "üblichen" Gruppen zuzusetzen. Erfindungsgemäß bevorzugte Vorrichtungen sind daher dadurch gekennzeichnet, daß das Polyurethan-Polymer keine inerten Füllstoffe aus der Gruppe der Tone, Carbonate und Titandioxid enthält.
In ganz besonders bevorzugten erfindungsgemäßen Vorrichtungen enthält das thermoplatische Polyurethan-Elastomer keinerlei inerte Füllstoffe.

Die erfindungsgemäße Vorrichtung umfaßt ein Haarteil aus jeweils mehreren Natur- und/oder Kunsthaaren sowie ein am Haarteil angebrachtes Befestigungselement, wobei das Befestigungselement mindestens ein thermoplatische Polyurethan-Elastomer umfaßt. Besonders bevorzugte erfindungsgemäße Vorrichtungen nutzen einen hohen Anteil an Polyurethanen im Befestigungselement. Hier sind Vorrichtungen bevorzugt, bei denen das Befestigungselement - bezogen auf das Gewicht des Befestigungselementes - mindestens 10 Gew.-%, vorzugsweise mindestens 25 Gew.-%, weiter bevorzugt mindestens 50 Gew.-%, noch weiter bevorzugt mindestens 75 Gew.-% und insbesondere mindestens 90 Gew.-% thermoplatische(s) Polyurethan-Elastomer(e) enthält. Ganz besonders bevorzugte erfindungsgemäße Vorrichtungen sind dadurch gekennzeichnet, daß das Befestigungselement ausschließlich aus thermoplatische(m/n) Polyurethan-Elastomer(en) besteht.

Erfindungsgemäß besonders bevorzugte polyesterbasierte thermoplatische Polyurethan-Elastomere sind kommerziell erhältlich. Besonders geeignet sind beispielsweise
- Merquinsa Pearlstick^{®} 46-10/03 Polyester based TPU
- Merquinsa Pearlstick^{®} 46-10/06 Polyester based TPU
- Merquinsa Pearlstick^{®} 46-10/12 Polyester based TPU
- Merquinsa Pearlstick^{®} 46-10/16 Polyester based TPU
- Merquinsa Pearlstick^{®} 46-73/15 Polyester based TPU
- Merquinsa Pearlstick^{®} 46-73/19 Polyester based TPU
- Merquinsa Pearlstick^{®} 46-73/27 Polyester based TPU
- Merquinsa Pearlstick^{®} 46-73/32 Polyester based TPU
- Merqulnsa Pearlstick^{®} 49-46/15 Polyester based TPU
- Merquinsa Pearlstick^{®} 49-46/19 Polyester based TPU

Diese weisen bei 20°C in 15 Gew.-%-iger Lösung In Methylethylketon (Brookfield-Viskosimeter RVF, Spindel 2, 20 U/min) folgende Viskositäten auf:

| | |
|---|---|
| 46-10/03 | 200-400 |
| 46-10/06 | 450-750 |
| 46-10/12 | 1000-1400 |
| 46-10/16 | 1400-1800 |
| 46-73/15 | 1300-1700 |
| 46-73/19 | 1700-2100 |
| 46-73/27 | 2500-2900 |
| 46-73/32 | 3000-3500 |
| 49-46/15 | 1300-1700 |
| 49-46/19 | 1700-2100 |

In der einfachsten Variante der erfindungsgemäßen Vorrichtungen werden einzelne Haarsträhnen mit jeweils einem Befestigungselement bereitgestellt und jede Haarsträhnen für sich am Eigenhaar befestigt.

Es ist erfindungsgemäß aber auch möglich, eine Mehrzahl von Strähnen gleichzeitig zu applizieren. Dies läßt sich bequem realisieren, indem mehrere Strähnen mit ihren Befestigungselementen auf ein Trägermaterial (z.B. einem Filmstreifen) aufgebracht werden und mehrere Befestigungselemente auf dem Träger lokalisiert sind. Auf diese Welse werden in einem Applikationsschritt am Eigenhaar an verschiedenen Stellen Haarsträhnen angebracht.

Eine solche erfindungsgemäßen Vorrichtungen von Haarsträhnen bestehen aus einer Anzahl von Haarsträhnen, die vorzugsweise fluchtend und im Wesentlichen parallel angeordnet sind, und die aus einer Anzahl von im Wesentlichen vorbestimmten Haaren bestehen, die natürlich oder künstlich sein können und geeignet ausgewählte Farben aufweisen, und zwar einfarbig oder mit Strähnchen. Die Haarsträhnen werden vorzugsweise im Wesentlichen in gleichen Abständen angeordnet. Sie erstrecken sich von ihren jeweiligen Enden mit den Befestigungselementen, die dazu ausgestaltet sind, mit dem Kopfhaar verbunden zu werden, bis hin zu ihren freien Enden.

Eine solche Vorrichtung umfaßt ebenfalls ein Trägerband, auf der die Befestigungselemente angeordnet sind.

Der Werkstoff, der das Trägerband bildet, ist vorzugsweise dünn und filmartig. Er kann nach der Verdampfung des Lösungsmittels aus dem Eigenhaar entfernt werden, was jedoch unangenehmes "Ziepen" zur Folge haben kann. Bevorzugt sind Trägerbänder, die so dünn und unauffällig sind, daß sie im Haar verbleiben können, ohne das Tragegefühl zu stören oder optisch aufzufallen.

In einer bevorzugten Ausführungsform dieser Variante besteht das Trägerband aus saugfähigem Material.

Bevorzugte erfindungsgemäße Vorrichtungen sind **dadurch gekennzeichnet, daß** die mit einem Befestigungselement versehene(n) Haarsträhne(n) zusätzlich ein Trägerband aufweist/aufweisen, das aus saugfähigem Material besteht.

Das Trägerband kann bei dieser Variante eine definierte Länge aufweisen und nur eine einzelne oder eine vorbestimmte Anzahl von Haarsträhnen tragen, oder es kann eine undefinierte Länge aufweisen, so dass die ausführende Person Abschnitte abschneiden kann, um die gewünschte Anzahl von Haarsträhnen zu erhalten. In allen Fällen kann die ausführende Person eine oder mehrere Haarsträhnen, die mit der aktuellen Anbringung nicht kompatibel sind, entfernen, indem sie einfach einen Teil des Trägerbands abschneidet. Auf jeder Anordnung können die Haarsträhnen die gleiche Länge, Größe und Farbe aufweisen, es ist aber auch möglich, Variationen in der Länge vorzusehen, um stufige Verlängerungen herzustellen, sowie Farbvariationen, um komplexe Strähnen und unterschiedliche Größen erzeugen zu können.

Eine bevorzugte Variante der erfindungsgemäßen Vorrichtung ist **dadurch gekennzeichnet, daß** mehrere Haarsträhnen bereitgestellt werden, die fluchtend und im Wesentlichen parallel mit ihren Befestigungselementen auf einem Trägerband angebracht sind.

Wie bereits erwähnt, sind auch hier Varianten der erfindungsgemäßen Vorrichtung bevorzugt, bei denen das Trägerband aus saugfähigem Material besteht.

Besonders bevorzugte erfindungsgemäße Vorrichtungen sind **dadurch gekennzeichnet, daß** das Trägerband aus saugfähigem Papier, vorzugsweise aus Filterpapier, besteht.

Ein weiterer Gegenstand der vorliegenden Erfindung ist ein Verfahren zur Haarverlängerung, bei dem mindestens eine erfindungsgemäße Vorrichtung durch
- Erwärmen des Befestigungselementes,
- Anbringen an (jeweils) einer Haarsträhne des Eigenhaares und
- Erkaltenlassen
mit dem Eigenhar verbunden wird.

Ein weiterer Gegenstand der vorliegenden Erfindung ist ein Verfahren zur Haarverlängerung, bei dem mindestens eine erfindungsgemäße Vorrichtung 18 durch
- Aufquellen des Befestigungselementes mit Hilfe eines Lösungsmittels,
- Anbringen an (jeweils) einer Haarsträhne des Eigenhaares und
- Verdampfenlassen des Lösungsmittels
mit dem Eigenhaar verbunden wird.

Die erfindungsgemäßen Verfahren umfassen das Anbringen mindestens einer, vorzugsweise mehrerer erfindungsgemäßer Vorrichtungen an das Eigenhaar einer Kundin, wobei das Befestigungselement mit Hilfe von Wärme und/oder durch Lösungsmittel "aktiviert" wird. Lösungsmittelbasierte Verfahren nutzen vorzugsweise Lösungsmittel aus bestimmten Gruppen. Hier sind erfindungsgemäße Verfahren bevorzugt, bei denen das Lösungsmittel ausgewählt wird aus Aceton (DMK), Isopropanol, Hexan, Methylethylketon (MEK), Ethylacetat.

Unabhängig davon, ob das Befestigungselement mit Hilfe von Wärme und/oder durch Lösungsmittel "aktiviert" wird, erfolgt die Anbringung der erfindungsgemäßen Vorrichtung (Haarsträhne) vorzugsweise dicht an der Kopfhaut. Hier sind Verfahren bevorzugt, bei denen das Anbringen an (jeweils) einer Haarsträhne des Eigenhaares im Abstand von 0,5 bis 2 cm, vorzugsweise von 0,75 bis 1,5 cm und insbesondere von 0,9 bis 1,1 cm von der Kopfhaut erfolgt.

Das Anmodulieren der erfindungsgemäßen Vorrichtungen an Eigenhaarsträhnen ist mit den erfindungsgemäßen Vorrichtungen in sehr kurzer Zeit möglich. In bevorzugten erfindungsgemäßen Verfahren erfolgen Erkaltenlassen bzw. Verdampfenlassen des Lösungsmittels über einen Zeitraum von 10 bis 60 Minuten, vorzugsweise von 15 bis 45 Minuten und insbesondere von 20 bis 30 Minuten.

Ein weiterer Vorteil des erfindungsgemäßen Verfahrens besteht in der Reversibilität der Haarverlängerung. Wünscht die Kundin ihre Haarverlängerung nicht mehr, so muß die erfindungsgemäße Vorrichtung nicht - wie bei marktüblichen Systemen - durch den Friseur über die Erwärmung entfernt oder gar abgeschnitten werden,. Es ist vielmehr möglich, die Vorrichtungen problemlos aus dem Eigenhaar zu entfernen. Ein weiterer Gegenstand der vorliegenden Erfindung ist daher ein Verfahren zur Entfernung von Haarverlängerungen, bei dem mindestens eine mittels eines erfindungsgemäßen Verfahrens angebrachte Haarsträhne (erfindungsgemäße Vorrichtung) durch Einwirkung von Wärme und/oder Einwirkung mindestens eines Lösungsmittels auf das Befestigungselement vom Eigenhaar abgelöst wird.

## Patentansprüche

1. Vorrichtung zur Haarverlängerung, umfassend ein Haarteil aus jeweils mehreren Natur- und/oder Kunsthaaren sowie ein am Haarteil angebrachtes Befestigungselement, **dadurch gekennzeichnet, daß** das Befestigungselement mindestens ein themoplastisches Poiyurethan-Elastormer umfaßt, das zusammengesetzt ist aus:
a) mindestens einem Polyisocyanat,
b) mindestens einem Polyesterglykol.

2. Vorrichtung nach Anspruch 1, **dadurch gekennzeichnet, daß** das themoplastische Polyurethan-Elastormer als Monomer a) (Polyisocyanat) mindestens ein aromatisches Diisocyanat enthält, vorzugsweise ein Diisocyanat aus der Gruppe Toluoldiisocyanat, 4,4'-Diphenylmethandiisocyanat und 2,4"-Diphenylmethandiisocyanat sowie deren Mischungen.

3. Vorrichtung nach einem der Ansprüche 1 oder 2, **dadurch gekennzeichnet, daß** das themoplastische Polyurethan-Elastormer als Monomer b) (Polyesterglycol) mindestens eine Verbindung der Formel PEG-I) 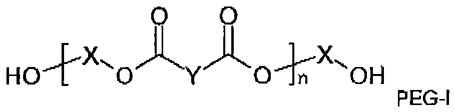 enthält, in der
- X für -(CH₂)ₘ- mit m = 2, 3, 4, 5, 6 oder für -(CH₂)ₖ-(C₆H₄)-(CH₂)ₖ- mit k = 1, 2, 3, 4, 5, 6 steht,
- Y für -(CH₂)ₘ- mit m = 0, 1, 2, 3, 4, 5, 6, 7, 8, 9, 10, 11, 12, 14, 16 oder für - (CH₂)ₖ-(C₆H₄)-(CH₂)ₖ- mit k = 1, 2, 3, 4, 5, 6 steht
- n eine ganze Zahl ist.

4. Vorrichtung nach einem der Ansprüche 1 bis 3, **dadurch gekennzeichnet, daß** das themoplastische Polyurethan-Elastormer als Monomer b) (Polyesterglycol) mindestens eine Verbindung der Formel PEG-II) 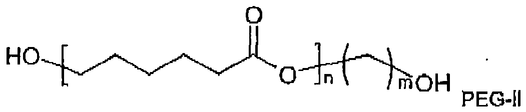 enthält, in der
- n für eine ganze Zahl steht,
- m für 2, 3, 4, 5, 6 steht

5. Vorrichtung nach einem der Ansprüche 1 bis 4, **dadurch gekennzeichnet, daß** das themoplastische Polyurethan-Elastormer Struktureinheiten der Formel 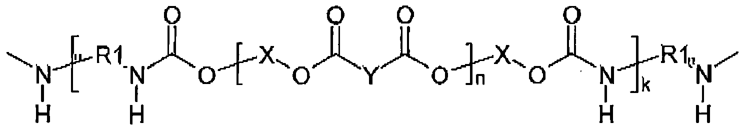 aufweist, in der
- X für -(CH₂)ₘ- mit m = 2, 3, 4, 5, 6 oder für -(CH₂)ₖ-(C₆H₄)-(CH₂)ₖ- mit k = 1, 2, 3, 4, 5, 6 steht,
- Y für -(CH₂)ₘ- mit m = 0, 1, 2, 3, 4, 5, 6, 7, 8, 9, 10, 11, 12, 14, 16 oder für-(CH₂)ₖ-(C₆H₄)-(CH₂)ₖ- mit k = 1, 2, 3, 4, 5, 6 steht
- R1 ausgewählt ist aus -(CH₂)₆-, 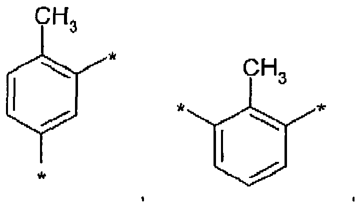 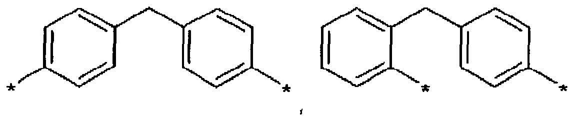
- n für eine ganze Zahl steht,
- k für eine ganze Zahl steht.

6. Vorrichtung nach einem der Ansprüche 1 bis 5, **dadurch gekennzeichnet, daß** das themoplastische Polyurethan-Elastormer Struktureinheiten der Formel 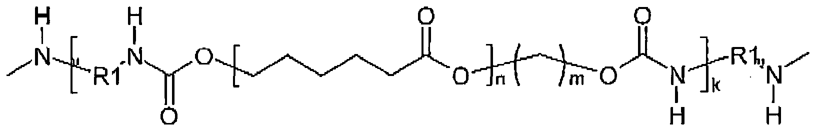 aufweist, in der
- R1 ausgewählt ist aus -(CH₂)₆-, 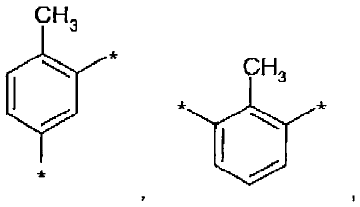 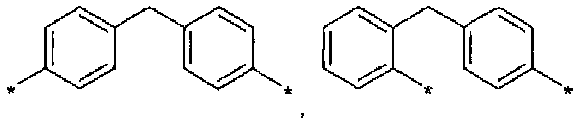
- n für eine ganze Zahl steht,
- m für 2, 3, 4, 6, 6 steht,
- k für eine ganze Zahl steht

7. Vorrichtung nach einem der Ansprüche 1 bis 6, **dadurch gekennzeichnet, daß** das themoplastische Polyurethan-Elastormer zusätzlich mindestens eine Struktureinheit der Formel
-[O-(CH₂)ₙ-O]-
Aufweist, in der n für 2, 3, 4, 5, oder 6 steht.

8. Vorrichtung nach einem der Ansprüche 1 bis 7, **dadurch gekennzeichnet, daß** die Molekulargewichte der Polyesterglykole zwischen 1 500 und 30 000, vorzugsweise zwischen 2 500 und 6 000 liegen.

9. Vorrichtung nach einem der Ansprüche 1 bis 8, **dadurch gekennzeichnet, daß** das themoplastische Polyurethan-Elastormer bei 20°C in 15 Gew.-%-iger Lösung in Methylethylketon (Brookflied-Viskosimeter RVF, Spindel 2, 20 U/min) Viskositäten von 1000 bis 2500 mPas, vorzugsweise von 1200 bis 2000 mPas und insbesondere von 1400 bis 1800 mPas, aufweist.

10. Vorrichtung nach einem der Ansprüche 1 bis 9, **dadurch gekennzeichnet, daß** das themoplastische Polyurethan-Elastormer keine inerten Füllstoffe aus der Gruppe der Tone, Carbonate und Titandioxid enthält.

11. Vorrichtung nach einem der Ansprüche 1 bis 10, **dadurch gekennzeichnet, daß** das themoplastische Polyurethan-Elastormer keinerlei inerte Füllstoffe enthält.

12. Vorrichtung nach einem der Ansprüche 1 bis 11, **dadurch gekennzeichnet, daß** das Befestigungselement - bezogen auf das Gewicht des Befestigungselementes - mindestens 10 Gew.-%, vorzugsweise mindestens 25 Gew.-%, weiter bevorzugt mindestens 50 Gew.-%, noch weiter bevorzugt mindestens 75 Gew.-% und insbesondere mindestens 90 Gew.-% Polyurethan-Polymer(e) enthält.

13. Vorrichtung nach einem der Ansprüche 1 bis 12, **dadurch gekennzeichnet, daß** das Befestigungselement ausschließlich aus themoplastische Polyurethan-Elastormer(en) besteht.

14. Vorrichtung nach einem der Ansprüche 1 bis 13, **dadurch gekennzeichnet, daß** die mit einem Befestigungselement versehene(n) Haarsträhne(n) zusätzlich ein Tragerband aufweist/aufweisen, das aus saugfähigem Material besteht.

15. Vorrichtung nach einem der Anspruche 1 bis 14, **dadurch gekennzeichnet, daß** mehrere Haarsträhnen bereitgestellt werden, die fluchtend und im wesentlichen parallel mit ihren Befestigungselementen auf einem Trägerband angebracht sind.

16. Vorrichtung nach Anspruch 15, **dadurch gekennzeichnet, daß** das Trägerband aus saugfähigem Material besteht.

17. Vorrichtung nach einem der Ansprüche 14 bis 16, **dadurch gekennzeichnet, daß** das Trägerband aus saugfähigem Papier, vorzugsweise aus Filterpapier, besteht.

18. Verfahren zur Haarverlängerung und/oder Haarverdichtung, **dadurch gekennzeichnet, daß** mindestens eine Vorrichtung nach einem der Ansprüche 1 bis 17 durch
- Erwärmen des Befestigungselementes,
- Anbringen an (jeweils) einer Haarsträhne des Eigenhaares und
- Erkaltenlassen
mit dem Eigenhaar verbunden wird.

19. Verfahren zur Haarverlängerung und/oder Haarverdichtung, **dadurch gekennzeichnet, daß** mindestens eine Vorrichtung nach einem der Ansprüche 1 bis 17 durch
- Aufquellen des Befestigungselementes mit Hilfe eines Lösungsmittels,
- Anbringen an (jeweils) einer Haarsträhne des Eigenhaares und
- Verdampfenlassen des Lösungsmittels
mit dem Eigenhaar verbunden wird.

20. Verfahren nach Anspruch 19, **dadurch gekennzeichnet, daß** das Lösungsmittels ausgewählt wird aus Aceton (DMK), Isopropanol, Hexan, Methylethylketon (MEK), Methylacetat

21. Verfahren nach einem der Ansprüche 18 bis 20, **dadurch gekennzeichnet, daß** das Anbringen an (jeweils) einer Haarsträhne des Eigenhaares im Abstand von 0,5 bis 2 cm, vorzugsweise von 0,75 bis 1,5 cm und insbesondere von 0,9 bis 1,1 cm von der Kopfhaut erfolgt.

22. Verfahren nach einem der Ansprüche 18 bis 21, **dadurch gekennzeichnet, daß** Erkaltenlassen bzw. Verdampfenlassen des Lösungsmittels über einen Zeitraum von 10 bis 60 Minuten, vorzugsweise von 15 bis 45 Minuten und insbesondere von 20 bis 30 Minuten, erfolgt.

23. Verfahren zur Entfernung von Haarverlängerungen, **dadurch gekennzeichnet, daß** mindestens eine mittels eines Verfahren nach einem der Ansprüche 18 bis 22 angebrachte Haarsträhne durch Einwirkung von Wärme und/oder Einwirkung mindestens eines Lösungsmittels auf das Befestigungselement vom Eigenhaar abgelöst wird.

## Claims

1. A device for extending hair, comprising a hair piece made in each case of multiple natural and/or synthetic hairs and an attachment element provided on the hair piece, **characterized in that** the attachment element comprises at least one thermoplastic polyurethane elastomer, which is composed of:
a) at least one polyisocyanate, and
b) at least one polyester glycol.

2. The device according to claim 1, **characterized in that** the thermoplastic polyurethane elastomer contains at least one aromatic diisocyanate as monomer a) (polyisocyanate), preferably a diisocyanate from the group toluene diisocyanate, 4,4'-diphenylmethane diisocyanate, and 2,4"-diphenylmethane diisocyanate, and the mixtures thereof.

3. The device according to either claim 1 or 2, **characterized in that** the thermoplastic polyurethane elastomer contains at least one compound of formula PEG-I) as monomer b) (polyester glycol), 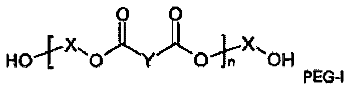 in which
- X denotes -(CH₂)ₘ- where m = 2, 3, 4, 5, 6 or -(CH₂)ₖ-(C₆H₄)-(CH₂)ₖ- where k = 1, 2, 3, 4, 5, 6;
- Y denotes -(CH₂)ₘ- where m = 0, 1, 2, 3, 4, 5, 6, 7, 8, 9, 10, 11, 12, 14, 16 or -(CH₂)ₖ-(C₆H₄)-(CH₂)ₖ- where k = 1, 2, 3, 4, 5, 6; and
- n is an integer.

4. The device according to one of claims 1 to 3, **characterized in that** the thermoplastic polyurethane elastomer contains at least one compound of formula PEG-II) as monomer b) (polyester glycol), 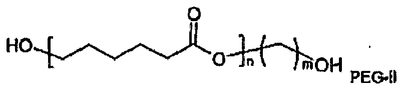 in which
- n denotes an integer, and
- m denotes 2, 3, 4, 5, 6.

5. The device according to one of claims 1 to 4, **characterized in that** the thermoplastic polyurethane elastomer comprises structural units of the formula 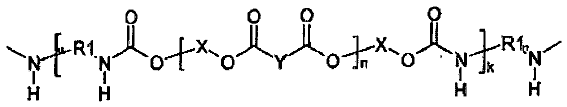 in which
- X denotes -(CH₂)ₘ- where m = 2, 3, 4, 5, 6 or -(CH₂)ₖ-(C₆H₄)-(CH₂)ₖ- where k = 1, 2, 3, 4, 5, 6;
- Y denotes -(CH₂)ₘ- where m = 0, 1, 2, 3, 4, 5, 6, 7, 8, 9, 10, 11, 12, 14, 16 or -(CH₂)ₖ-(C₆H₄)-(CH₂)ₖ- where k = 1, 2, 3, 4, 5, 6;
- n is an integer, 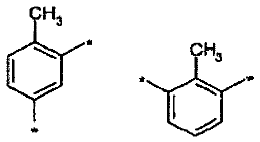
- R1 is selected from -(CH₂)₆-, 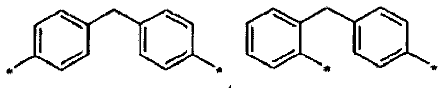
- n denotes an integer, and
- k denotes an integer.

6. The device according to one of claims 1 to 5, **characterized in that** the thermoplastic polyurethane elastomer comprises structural units of the formula 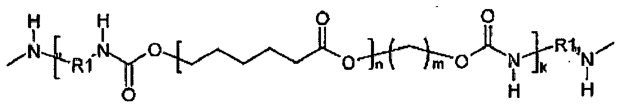 in which 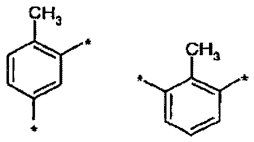
- R1 is selected from -(CH₂)₆-, 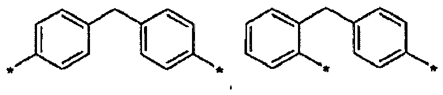
- n denotes an integer,
- m denotes 2, 3, 4, 5, 6, and
- k denotes an integer.

7. The device according to one of claims 1 to 6, **characterized in that** the thermoplastic polyurethane elastomer additionally comprises at least one structural unit of formula -[O-(CH₂)ₙ-O]-,
in which n denotes 2, 3, 4, 5 or 6.

8. The device according to one of claims 1 to 7, **characterized in that** the molecular weights of the polyester glycols range between 1,500 and 30,000, preferably between 2,500 and 6,000.

9. The device according to one of claims 1 to 8, **characterized in that** the thermoplastic polyurethane elastomer, at 20°C in a 15% by weight solution in methyl ethyl ketone (Brookfield viscometer RVF, spindle 2, 20 rpm), has viscosity levels from 1000 to 2500 mPas, preferably from 1200 to 2000 mPas, and in particular from 1400 to 1800 mPas.

10. The device according to one of claims 1 to 9, **characterized in that** the thermoplastic polyurethane elastomer does not contain any inert fillers from the group of clays, carbonates and titanium dioxide.

11. The device according to one of claims 1 to 10, **characterized in that** the thermoplastic polyurethane elastomer does not contain any inert fillers whatsoever.

12. The device according to one of claims 1 to 11, **characterized in that** the attachment element - based on the weight of the attachment element - contains at least 10 wt.%, preferably at least 25 wt.%, further preferably at least 50 wt.%, still more preferably at least 75 wt.%, and in particular at least 90 wt.% polyurethane polymer(s).

13. The device according to one of claims 1 to 12, **characterized in that** the attachment element consists exclusively of thermoplastic polyurethane elastomer(s).

14. The device according to one of claims 1 to 13, **characterized in that** the strand(s) of hair provided with an attachment element additionally comprise(s) a carrier tape made of absorbent material.

15. The device according to one of claims 1 to 14, **characterized in that** multiple strands of hair are supplied, which are provided with the attachment elements thereof aligned and substantially parallel on a carrier tape.

16. The device according to claim 15, **characterized in that** the carrier tape is made of absorbent material.

17. The device according to one of claims 14 to 16, **characterized in that** the carrier tape is made of absorbent paper, preferably filter paper.

18. A method for extending hair and/or thickening hair, **characterized in that** at least one device according to one of claims 1 to 17 is joined to the person's own hair by
- heating the attachment element,
- attaching it (in each case) to a strand of the person's own hair, and
- cooling.

19. A method for extending hair and/or thickening hair, **characterized in that** at least one device according to one of claims 1 to 17 is joined to the person's own hair by
- swelling the attachment element with the aid of a solvent,
- attaching it (in each case) to a strand of the person's own hair, and
- allowing the solvent to evaporate.

20. The method according to claim 19, **characterized in that** the solvent is selected from acetone (DMK), isopropanol, hexane, methyl ethyl ketone (MEK), and ethyl acrylate.

21. The method according to one of claims 18 to 20, **characterized in that** the attaching to a strand of the persons' own hair (in each case) takes place at a distance from 0.5 to 2 cm, preferably from 0.75 to 1.5 cm, and in particular from 0.9 to 1.1 cm from the scalp.

22. The method according to one of claims 18 to 21, **characterized in that** cooling or allowing the solvent to evaporate takes place over a time period from 10 to 60 minutes, preferably from 15 to 45 minutes, and in particular from 20 to 30 minutes.

23. A method for removing hair extensions, **characterized in that** at least one strand of hair attached by way of a method according to one of claims 18 to 22 is detached from the person's own hair by the action of heat and/or by the action of at least one solvent on the attachment element.

## Revendications

1. Dispositif d'allongement des cheveux, comprenant un postiche constitué de plusieurs cheveux naturels et/ou artificiels et un élément de fixation placé sur le postiche, **caractérisé en ce que** l'élément de fixation comporte au moins un élastomère de polyuréthanne thermoplastique, qui se compose de :
a) au moins un polyisocyanate,
b) au moins un polyester glycol.

2. Dispositif selon la revendication 1, **caractérisé en ce que** l'élastomère de polyuréthane thermoplastique contient comme monomère a) (polyisocyanate) au moins un diisocyanate aromatique, de préférence un diisocyanate du groupe comprenant le toluène, le 4,4'-diphénylméthane-diisocyanate et le 2,4"-diphénylméthane-diisocyanate et leurs mélanges.

3. Dispositif selon l'une des revendications 1 ou 2, **caractérisé en ce que** l'élastomère de polyuréthane thermoplastique contient monomère b) (polyester glycol) au moins un composé de la formule PEG-I : 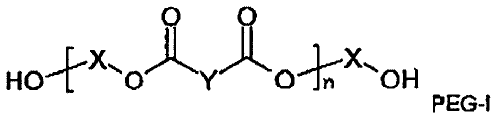 dans laquelle
- X représente -(CH₂)ₘ- avec m = 2, 3, 4, 5, 6, ou - {CH₂)ₖ-(C₆H₄)-(CH₂)ₖ avec k = 1 2, 3, 4, 5 , 6,
- Y représente -(CH₂)ₘ avec m = 0, 1, 2, 3, 4, 5, 6, 7, 8, 9, 10, 11, 12, 14, 16, ou -(CH₂)ₖ-(C₆H₄)-(CH₂)ₖ avec k = 1, 2, 3, 4, 5, 6,
- n est un nombre entier.

4. Dispositif selon l'une des revendications 1 à 3, **caractérisé en ce que** l'élastomère de polyuréthane thermoplastique contient comme monomère b) (polyester glycol) d'au moins un composé de la formule PEG-II : 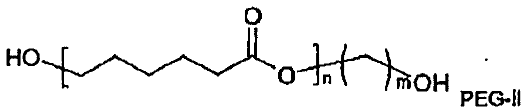 dans laquelle
n est un nombre entier,
m est égal à 2, 3, 4, 5, 6.

5. Dispositif selon l'une des revendications 1 à 4, **caractérisé en ce que** l'élastomère de polyuréthanne thermoplastique comportant des unités structurelles de la formule 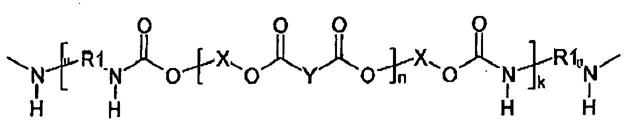 dans laquelle
- X représente -(CH₂)ₘ- avec m = 2, 3, 4, 5, 6, ou -(CH₂)ₖ-(C₆H₄)-(CH₂)ₖ- avec k = 1, 2,3,4,5,6,
- Y est -(CH₂)ₘ- avec m = 0, 1, 2, 3, 4, 5, 6, 7, 8, 9, 10, 11, 12, 14, 16, ou est -(CH₂)_{K}-(C₆H₄)-(CH₂)ₖ- avec k = 1, 2, 3, 4, 5, 6,
- R1 est choisi parmi -(CH₂)₆-, 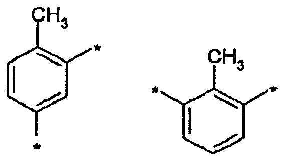 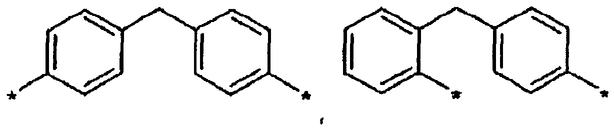
- n est un nombre entier,
- k est un nombre entier.

6. Dispositif selon l'une des revendications 1 à 5, **caractérisé en ce que** l'élastomère de polyuréthane thermoplastique comporte des unités structurelles de la formule 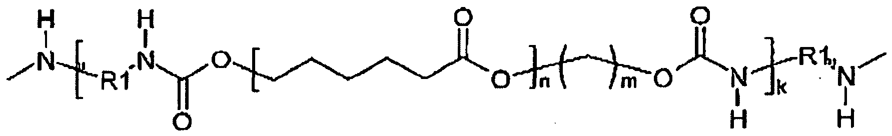 dans laquelle
- R1 est choisi parmi -(CH₂)₆-, 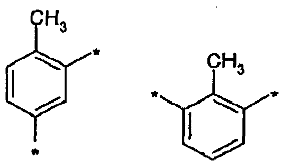 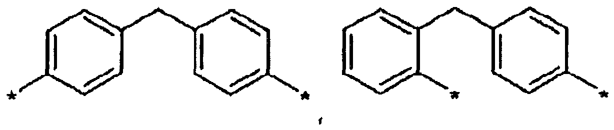
- n est un nombre entier,
- m est égal à 2, 3, 4, 5, 6,
- k est un nombre entier.

7. Dispositif selon l'une des revendications 1 à 6, **caractérisé en ce que** l'élastomère de polyuréthane thermoplastique comporte en outre au moins une unité structurale de la formule -[O-(CH₂)ₙ-O]-
dans laquelle n est égal à 2, 3, 4, 5 ou 6.

8. Dispositif selon l'une des revendications 1 à 7, **caractérisé en ce que** les poids moléculaires des polyester glycols sont compris entre 1 500 à 30 000, de préférence 2 500 à 6 000.

9. Dispositif selon l'une quelconque des revendications 1 à 8, **caractérisé en ce que** l'élastomère de polyuréthane thermoplastique présente, à 20°C dans une solution de 15% en poids de méthyléthyl-cétone (viscosimètre Brookflied RVF, broche 2, 20 tr/min), des viscosités allant de 1000 à 2500 mPas, de préférence de 1200 à 2000 mPas et en particulier de 1400 à 1800 mPas.

10. Dispositif selon l'une des revendications 1 à 9, **caractérisé en ce que** l'élastomère de polyuréthane thermoplastique ne contient pas de charges inertes dans le groupe comprenant des argiles, des carbonates et le dioxyde de titane.

11. Dispositif selon l'une des revendications 1 à 10, **caractérisé en ce que** l'élastomère de polyuréthane thermoplastique ne contient pas de charges inertes.

12. Dispositif selon l'une des revendications 1 à 11, **caractérisé en ce que** l'élément de fixation contient, sur la base du poids de l'élément de fixation, au moins 10% en poids, de préférence au moins 25% en poids, plus préférablement au moins 50% en poids, encore plus préférablement au moins 75% en poids en particulier au moins 90% en poids, de polymère(s) de polyuréthane.

13. Dispositif selon l'une des revendications 1 à 12, **caractérisé en ce que** l'élément de fixation est exclusivement constitué d'élastomère(s) de polyuréthanne thermoplastique(s).

14. Dispositif selon l'une des revendications 1 à 13, **caractérisé en ce que** la ou les mèches de cheveux, pourvue(s) d'un élément de fixation comprennent en outre une bande de support constituée d'une matière absorbante.

15. Dispositif selon l'une des revendications 1 à 14, **caractérisé en ce qu'**il est prévu plusieurs mèches de cheveux qui sont placées sur une bande de support en alignement et sensiblement parallèlement à leurs éléments de fixation.

16. Dispositif selon la revendication 15, **caractérisé en ce que** la bande de support est constituée d'une matière absorbante.

17. Dispositif selon l'une des revendications 14 à 16, **caractérisé en ce que** la bande de support est constituée de papier absorbant, de préférence de papier filtre.

18. Procédé d'allongement et/ou d'épaississement des cheveux, **caractérisé en ce qu'**on relie au moins un dispositif selon l'une des revendications 1 à 17 à la chevelure d'une personne
- en chauffant l'élément de fixation,
- en le plaçant (à chaque fois) sur une mèche de cheveux de la chevelure et
- en le laissant refroidir.

19. Procédé d'allongement et/ou d'épaississement des cheveux, **caractérisé en ce qu'**on relie au moins un dispositif selon l'une des revendications 1 à 17 à la chevelure d'une personne
- en faisant gonfler l'élément de fixation à l'aide d'un solvant,
- en le plaçant (à chaque fois) une mèche de cheveux de la chevelure et
- en laissant évaporer le solvant.

20. Procédé selon la revendication 19, **caractérisé en ce que** le solvant est choisi parmi l'acétone (DMK), l'isopropanol, l'hexane, le méthyléthyl-cétone (MEK), l'acétate d'éthyle.

21. Procédé selon l'une des revendications 18 à 20, **caractérisé en ce que** le placement (à chaque fois) sur une mèche de cheveux de la chevelure d'une personne est effectuée à une distance de 0,5 à 2 cm, de préférence de 0,75 à 1,5 cm et en particulier de 0,9 à 1,1 cm du cuir chevelu.

22. Procédé selon l'une des revendications 18 à 21, **caractérisé en ce qu'**on laisse refroidir et évaporer le solvant pendant une durée de 10 à 60 minutes, de préférence de 15 à 45 minutes et en particulier de 20 à 30 minutes.

23. Procédé de suppression de cheveux allongés, **caractérisé en ce qu'**on retirer au moins une mèche de cheveux, placée au moyen d'un procédé selon l'une des revendications 18 à 22, de la chevelure d'une personne en soumettant l'élément de fixation à l'action de la chaleur et/ou à l'action d'au moins un solvant.
